# EUROPEAN PATENT SPECIFICATION

(11) **EP 2 701 732 B1**
(45) Date of publication and mention of the grant of the patent: **30.08.2017**
(21) Application number: 11741681.8
(22) Date of filing: 08.07.2011
(51) Int. Cl.: A61K 38/45, A61K 9/00, A61K 48/00, C12N 9/10, A61P 1/16, A61P 3/06

(54) **LECITHIN:CHOLESTEROL ACYLTRANSFERASE (LCAT) AND ITS ROLE IN NONALCOHOLIC FATTY LIVER DISEASE (NAFLD)**
LECITHIN-CHOLESTERIN-ACYLTRANSFERASE (LCAT) UND SEINE ROLLE BEI NICHT-ALKOHOLISCHER FETTLEBERERKRANKUNG (NAFLD)
LÉCITHINE/CHOLESTÉROL ACYLTRANSFÉRASE (LCAT) ET SON RÔLE DANS LA STÉATOSE HÉPATIQUE NON ALCOOLIQUE (SHNA)

(30) Priority: 25.02.2011 GR 20110100124
(43) Date of publication of application: 05.03.2014
(73) Proprietor: University Of Patras, 26500 Rio Patras (GR); Kypreos, Kyriakos E., 26500 Agios Vassilios Patron (GR)
(72) Inventor: KYPREOS, Kyriakos E., GR-26500 Agios Vassilios Patron (GR); PAPACHRISTOU, Dionysios J., GR-26500 Rio (GR)
(74) Representative: Malamis, Alkisti-Irene
(86) International application number: PCT/GR2011/000026
(87) International publication number: WO 2012/114131

(56) References cited:
- WO-A1-2010/144904
- WO-A2-2009/015314
- ROUSSET X ET AL: "Lecithin cholesterol acyltransferase: An anti- or pro-atherogenic factor?", CURRENT ATHEROSCLEROSIS REPORTS 2011 CURRENT MEDICINE GROUP LLC GBR LNKD- DOI:10.1007/S11883-011-0171-6, vol. 13, no. 3, February 2011 (2011-02), pages 249-256, XP002659712, ISSN: 1523-3804
- ALGER HEATHER M ET AL: "Inhibition of Acyl-Coenzyme A: Cholesterol Acyltransferase 2 (ACAT2) Prevents Dietary Cholesterol-associated Steatosis by Enhancing Hepatic Triglyceride Mobilization", JOURNAL OF BIOLOGICAL CHEMISTRY, vol. 285, no. 19, May 2010 (2010-05), pages 14267-14274, XP002659713, ISSN: 0021-9258
- DULLAART ROBIN P F ET AL: "Plasma Lecithin: Cholesterol Acyltransferase Activity Is Elevated in Metabolic Syndrome and Is an Independent Marker of Increased Carotid Artery Intima Media Thickness", JOURNAL OF CLINICAL ENDOCRINOLOGY & METABOLISM, vol. 93, no. 12, December 2008 (2008-12), pages 4860-4866, XP002659714, ISSN: 0021-972X
- BRUNT E M ET AL: "Histopathology of nonalcoholic fatty liver disease", WORLD JOURNAL OF GASTROENTEROLOGY 2010 WJG PRESS CHN, vol. 16, no. 42, November 2010 (2010-11), pages 5286-5296, ISSN: 1007-9327

## Description

### Field of the invention

The invention describes lecithin:cholesterol acyltransferase (LCAT) as a novel therapeutic agent for use in the treatment of nonalcoholic fatty liver disease (NAFLD).

In the present description and claims, the following abbreviations are used:
ApoE, Apolipoprotein E; LCAT, lecithin: cholesterol acyltransferase; LCAT^{-/-}, lecithin:cholesterol acyltransferase deficient; GFP, Green fluorescent protein; Ad-LCAT, recombinant attenuated adenovirus expressing LCAT; AdGFP, recombinant attenuated adenovirus expressing GFP; BMI, Body-mass index; GTT, glucose tolerance test; HDL, high density lipoprotein; VLDL, very low density lipoprotein; LDL, low density lipoprotein; IST, insulin sensitivity test; NAFLD, nonalcoholic fatty liver disease; NASH, nonalcoholic steatohepatitis; pfu, plaque forming units; WT, wild-type.

### Prior art

Nonalcoholic fatty liver disease (NAFLD) is a spectrum of metabolic abnormalities ranging from a simple accumulation of triglycerides in the liver (hepatic steatosis) to hepatic steatosis with inflammation, fibrosis, and cirrhosis (nonalcoholic steatohepatitis or NASH) (1;2). Aging, hormonal imbalance, and genetic predisposition may contribute to NAFLD (3). However, western-type diet and sedentary lifestyle that result in excess body fat, physical inactivity and imbalance in caloric load, are the most common contributors (4).

Although hepatic steatosis may be related to a number of clinical disorders, in humans NAFLD is usually characterized by obesity and in some instances by insulin resistance and other metabolic perturbations (1;2;5). In one epidemiological study 48% of subjects diagnosed with metabolic syndrome were found to have fatty liver by ultrasound (6). Specifically, fatty liver was diagnosed in 39% of those with a body mass index (BMI) 25 kg/m² or greater, in 41% of those with known diabetes, and in 32% of those with dyslipidemia (6). In another multiethnic urban U.S. population study using magnetic resonance spectroscopy (MRS), the presence of elevated hepatic triglycerides was more frequent in those with obesity (BMI > 30 kg/m²) and metabolic syndrome (7). Thus, it has been proposed that NAFLD should be included as a component of metabolic syndrome (8).

Depending on the conditions, NAFLD may progress to nonalcoholic steatohepatitis (NASH). Studies in humans and animal models have suggested that alterations in hepatic lipid metabolism, increased generation of reactive oxygen species and consequently oxidative stress, changes in mitochondrial function, DNA damage, microbial infections and release of various cytokines may contribute to the progression of NAFLD to NASH (4;9-11).

One of the most important protein components of the lipoprotein metabolic system is lecithin:cholesterol acyltransferase (LCAT), a plasma enzyme that is produced and secreted mainly by the liver. LCAT catalyzes the esterification of free cholesterol of HDL and VLDL lipoproteins by transferring a fatty acyl group from the C-2 position of lecithin to the 3-hydroxyl group of cholesterol (12-15). The importance of LCAT in lipid and lipoprotein metabolism was first established with the discovery of three Scandinavian families deficient in the enzyme (16-23). All of the patients had abnormal plasma lipoproteins. Most notably, almost all plasma cholesterol was unesterified, while analysis of HDL by negative staining electron microscopy revealed the presence of stacks of discoidal HDL. More recent studies showed that patients with LCAT deficiency have both preβ-1 and α-4 HDL present in their plasma and develop corneal opacities, anemia, proteinuria, and kidney failure (24). VLDL and LDL from these patients were also abnormal in size and composition (23;25). Furthermore, all patients had reduced HDL cholesterol levels, and developed hypertriglyceridemia (16-23). In line with these observations, recently we reported that LCAT is a key enzyme in the metabolism of triglyceride-rich lipoproteins (26). Specifically, we found that adenovirus-mediated gene transfer of LCAT in mice ameliorated the hypertriglyceridemia triggered by apoE overexpression (26). Moreover, Brousseau *et al*., (27) suggested that LCAT activity contributes to LDL clearance and atheroprotection in an LDL receptor-dependent fashion.

In our previous studies we showed that the lipoprotein transport system is important in diet-induced obesity [28]. However, there is no hint whatsoever in the art and there is no reason to assume that LCAT modulates the severity of diet-induced NAFLD.

It is an object of the present invention to describe the role of LCAT in the development of NAFLD.

In the process of developing the present invention we used experimental mouse models. We report that deficiency in LCAT sensitizes mice to NAFLD and is associated with enhanced intestinal lipid absorption and accelerated clearance of postprandial lipids from the circulation and reduced rate of hepatic triglyceride secretion. Importantly, ectopic expression of human LCAT by adenovirus-mediated gene transfer in LCAT^{-/-} mice fed western-type diet for 12 weeks resulted in a significant reduction in their hepatic triglyceride content and a great improvement of hepatic histology and architecture compared to LCAT^{-/-} mice infected with a control adenovirus. The present invention as described in the specification, claims that LCAT is a novel therapeutic agent for the treatment of NAFLD.
The invention is illustrated by use if the following examples:

### MATERIALS AND METHODS

### Animal studies

The LCAT^{-/-} mice used in our studies were kindly donated to us by Dr. Silvia Santamarina-Fojo (28) and the C57BL/6 mice were purchased from Jackson Labs (Bar Harbor, Maine, USA). Male mice 10-12 weeks old were used in these studies. Mice in each group were caged individually (one mouse per cage) and were allowed unrestricted access to food and water. To ensure similar average cholesterol triglyceride and glucose levels and starting body weights, groups of 10 mice were formed after determining the fasting cholesterol, triglyceride and glucose levels and body weights of the individual mice. Mice were fed the standard western-type diet (Mucedola SRL, Milano, Italy) for the indicated period, and body weight and fasting plasma cholesterol and triglyceride levels were determined at the indicated time-points after diet initiation. At the end of each experiment, mice were sacrificed and plasma and liver samples were collected. Carcasses were stored at -80°C. The administration of recombinant attenuated adenoviruses to experimental mice, was performed as described previously (29;30). All animal studies were governed by the EU guidelines of the *Protocol for the Protection and Welfare of Animals.* In our experiments we took into consideration the 3Rs (reduce, refine, replace) and we minimized the number of animal experiments to the absolute minimum. To this date there is no *in vitro* system to mimic satisfactorily the lipid and lipoprotein transport system and the *in vivo* mechanisms leading to nonalcoholic fatty liver disease, making the use of experimental animals mandatory. All procedures used in our studies involve minimal distress to the mice tested. The work was authorized by the appropriate committee of the Laboratory Animal Center of The University of Patras Medical School.

### Amplification and expansion of recombinant adenoviruses

The amplification and expansion of the attenuated recombinant adenoviruses AdGFP and Ad-LCAT have been described previously (29). Briefly infection of confluent cultures of 293 cells in triple flasks (Nunc^{®}, Rochester, NY, USA) was followed by a purification process involving CsCl ultracentrifugation performed twice, followed by dialysis and titration of the virus. Typically, titers of approximately 2x10¹⁰ pfu/ml were obtained for each virus preparation.

### Plasma lipid determination

Following a 16 hour fasting period, plasma samples were isolated from the experimental mice. Plasma cholesterol, triglyceride and free-fatty acid levels were measured using the Cholesterol Kit (Thermo Fisher Scientific, Waltham, MA., USA), Triglyceride Determination Kit (Sigma, St. Louis, MO, USA) and NEFA C kit (Wako diagnostics, Richmond, VA., USA) respectively, according to the manufacturer's instructions and as described previously (29).

### Fractionation of plasma lipoproteins by density gradient ultracentrifugation

For the determination of plasma cholesterol and triglyceride levels in various plasma lipoproteins, 0.5 ml of pools of plasma from five LCAT^{-/-} and five C57BL/6 mice were fractionated by density gradient ultracentrifugation over a 10 ml KBr density gradient in a Beckmann Ultracentrifuge using an SW41 rotor, as described previously (29). The cholesterol and triglyceride content of different density fractions were determined as described above.

### Measurement of hepatic triglyceride content

For hepatic triglyceride determination, a liver sample was collected, weighed and then dissolved in 0.5 ml of 5M KOH in 50% ethanol solution by overnight incubation at 65°C. The *pH* of the solution was adjusted to 7, and the final volume was recorded. Total amount of triglycerides was determined in the resulting mixture as described above. Results are expressed as milligram (mg) of triglycerides per gram of tissue ± standard error of the mean.

### Histological analysis of tissue samples

At the end of each experiment, mice were sacrificed and liver and visceral fat specimens were collected and stored either at -80°C or fixed in 10% formalin. Four µm-thick sections were obtained from the formalin-fixed paraffin-embedded tissue for further histological analyses. Conventional H&E histological staining was performed in order to evaluate the microscopic morphology of the liver tissue samples. In order to assess the tissue structural integrity and architecture, the reticular fiber network was outlined with application of the reticulin stain according to the manufacturer's instructions (Bioptica, Milan, Italy). Stained sections were visualized using Olympus BX41 bright-field microscope. Histomorphometry was performed using Adobe Photoshop software. Specifically, 5 representative hepatic sections from each animal were used for histomorphometric measurements. Each section was photographed using a Nikon Eclipse 80i microscope with a Nikon DXM 1200C digital camera (original magnification 40X). The digital images were imported into Adobe Photoshop CS2 and grid was added. For each section the number of lipid vacuoles that were intersected by the grid was determined and calculated independently by one pathologist (D.J.P.) and one investigator (K.E.K) in a blinded fashion. These data were then used to assess the total number of fat vacuoles accumulated within hepatocytes of each section. Conventional immunohistochemistry was performed as described previously (31) using an anti-CD68 mouse monoclonal antibody (dilution 1:70, # M 0718, DAKO, Denmark). Oil-red O staining of hepatic sections was performed for the visualization of the unsaturated hydrophobic lipids according to the manufacturer instructions (Oil Red O Stain kit, #RRSK14, Biostain Ready Reagents, Manchester, UK),

### Determination of daily food consumption

Food intake was assessed by determining the difference in food weight during a 7-day period to ensure reliable measurements, as described previously (32;33). Each day, the daily average food consumption of each group was determined and the average daily food consumption per mouse was calculated for each mouse strain. Results were then expressed as average food consumption per mouse per strain over the seven day period ± standard error of the mean.

### Determination of post-prandial triglyceride kinetics following oral administration of olive oil

Groups of 5 LCAT^{-/-} or C57BL/6 mice were tested. Prior to the experiment, mice were fasted overnight for 16 hours. The following day animals were administered an oral load of 0.5 ml of olive oil and plasma samples were isolated 30, 60, 120, 180, and 240 minutes following olive oil administration. A control sample for baseline triglyceride determination was isolated 1 minute prior to gavage administration of olive oil. Triglyceride levels were quantified in plasma samples as described above, and then plotted in graphs as a function of time. Values were expressed in mg/dl ± standard error of the mean.

### Rate of intestinal secretion of triglyceride-rich chylomicrons and hepatic VLDL triglyceride production

To determine the rate of intestinal triglyceride secretion in the plasma of our experimental mice we measured the total rate of plasma triglyceride input (intestinal and hepatic) and then subtracted the rate of hepatic triglyceride secretion.

Briefly, to determine the total rate of triglyceride input in the plasma of mice, groups of 5 LCAT^{-/-} or C57BL/6 mice were tested. Prior to the experiment, mice were fasted overnight for 16 hours. The following day animals were gavaged with 0.3 ml of olive oil and placed back to their cages for one hour (in our experimental setup dietary triglyceride absorption measured as a post-gavage increase in plasma triglyceride levels, becomes apparent at ∼1 hour following oral administration of olive oil). Immediately after, mice were injected with Triton-WR1339 at a dose of 500 mg per kg of body weight, using a 15% solution (w/v) in 0.9% NaCl (Triton-WR1339 has been shown to completely inhibit catabolism of triglyceride-rich lipoproteins (34)), as described previously (29;30;35;36). Then, serum samples were isolated at 30 min, 60 min, 90 min, 120 min, 150, and 180 min, after injection with Triton-WR1339. As baseline control, serum samples were isolated approximately 1 min after injection with the detergent. Plasma triglyceride levels at each time-point were determined as described above and linear graphs of triglyceride concentration *vs*. time were generated. The rate of plasma-triglyceride accumulation expressed in mg/dl/min was calculated from the slope of the linear graphs. Then, slopes were reported as mean ± standard error of the mean. Total plasma triglyceride supply equals to the sum of intestinal and hepatic triglyceride secretion.

To measure the rate of hepatic VLDL triglyceride secretion, groups of 4-6 LCAT^{-/-} and C57BL/6 mice were injected with Triton-WR1339 (Sigma, St. Louis, MO, USA) at a dose of 500 mg per kg of body weight, using a 15% solution (w/v) in 0.9% NaCl, as described above. Serum samples were isolated at 10 min, 20 min, 30 min, 40 min, 50 min and 60 min, after injection with Triton WR 1339. As control, serum samples were isolated approximately 1 min after injection with the detergent. Plasma triglyceride levels at each time-point were determined as described above and linear graphs of triglyceride concentration *vs*. time were generated. The rate of VLDL-triglyceride secretion expressed in mg/dl/min was calculated from the slope of the linear graphs. Then, slopes were reported as mean ± standard error of the mean.

Subtraction of the rate of hepatic triglyceride secretion from the total plasma triglyceride input yielded the rate of intestinal secretion of triglyceride-rich chylomicrons following an oral fat load, expressed as mean ± standard error of the mean.

### Statistical analysis

Comparison of data from two groups of mice was performed using the Student t-test. Where more than a two group comparison was required the results were analyzed using ANOVA. Data are reported as mean ± standard error of the mean. * indicates p<0.05 and ** indicates p<0.005

### Description of the Figures

The invention is illustrated by use of the following figures:

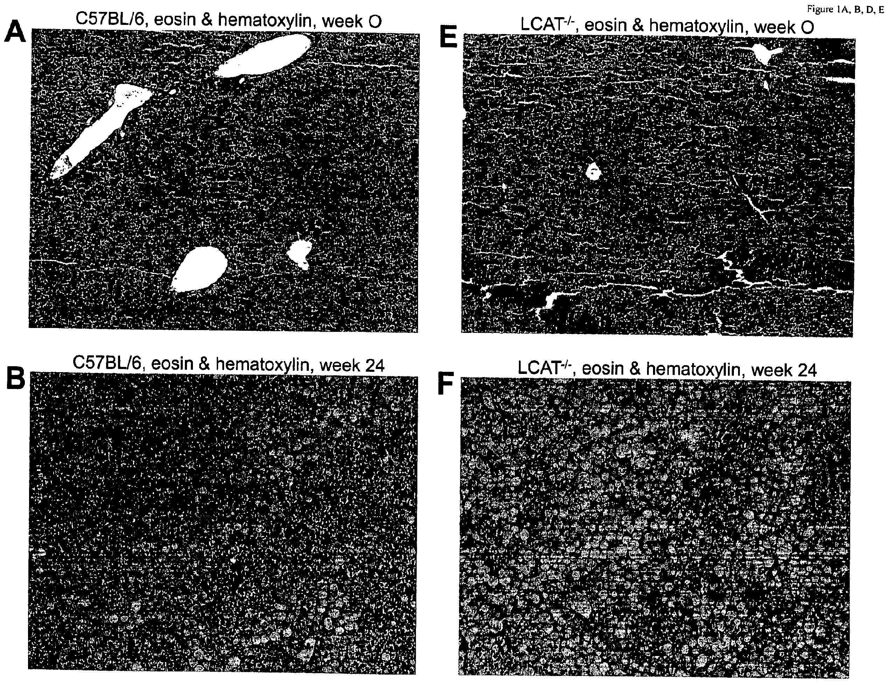

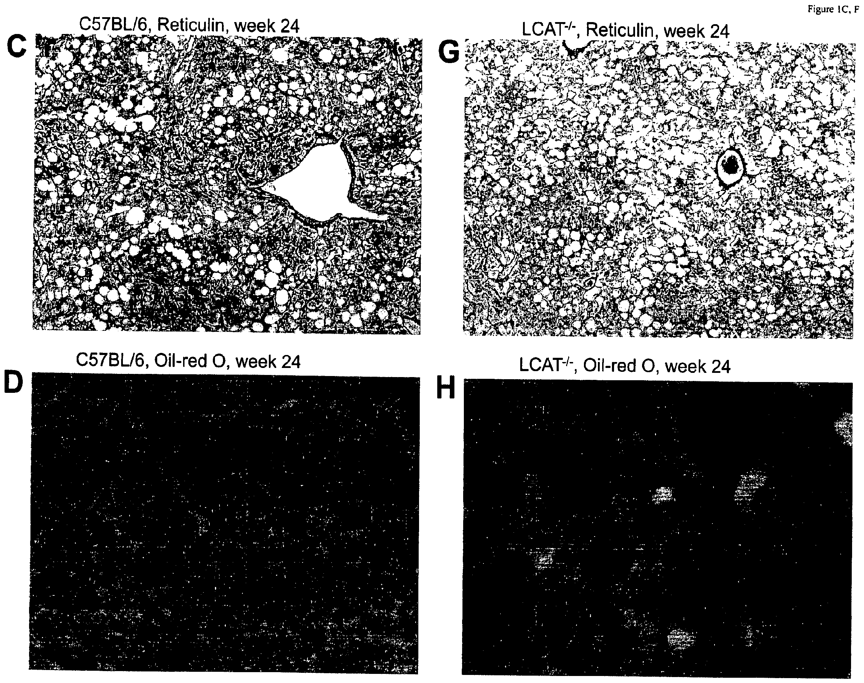

**Figure 1** shows the histological analyses of liver sections from LCAT^{-/-} and C57BL/6 mice. *Panels A, B, E, and F* are representative pictures of eosin/hematoxylin stained hepatic sections from C57BL/6 (*A, B*) and LCAT^{-/-} mice (*E, F*) at week 0 (*A, E*) and 24 (*B, F*) on western-type diet. *Panels C and G* show representative pictures of reticulin stained hepatic sections of C57BL/6 (*C*) and LCAT^{-/-} mice (*G*) fed western-type diet for 24 weeks. *Panels D and H* are representative pictures of oil-red O stained hepatic sections from C57BL/6 *(D)* or LCAT^{-/-} (*H*) mice that were fed western-type diet for 24 weeks. All pictures were taken under original magnification 40X.

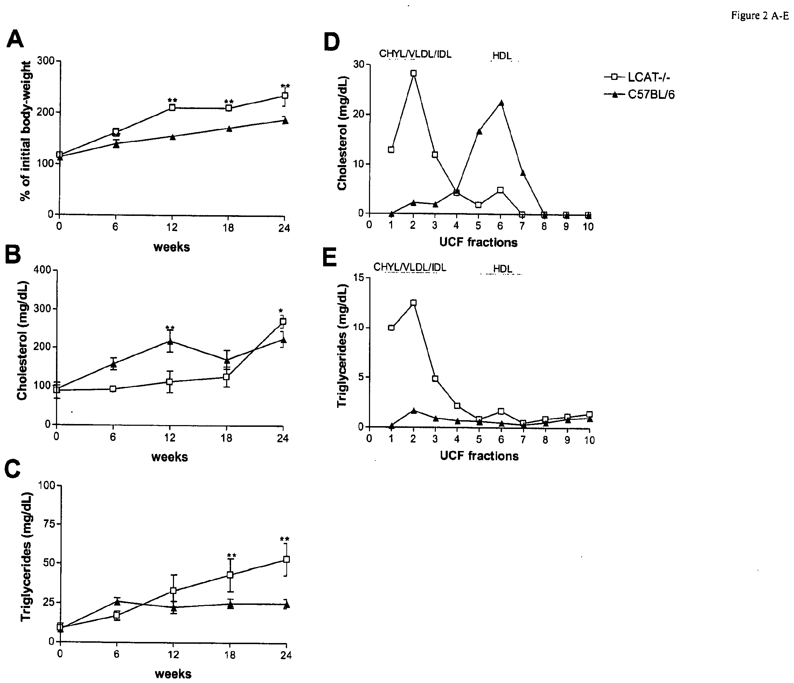

**Figure 2** shows biochemical parameters of LCAT^{-/-} and C57BL/6 mice fed western-type diet for a period of 24 weeks. Changes in (*A*) body weight, (*B*) plasma cholesterol, and (*C*) plasma triglycerides of mice fed western-type diet for 24 weeks. Panels (*D*) and (*E*) show the cholesterol and triglyceride content respectively, of the different density lipoprotein fractions following separation of plasma lipoproteins by density gradient ultracentrifugation (UCF). The different lipoprotein fractions are indicated.

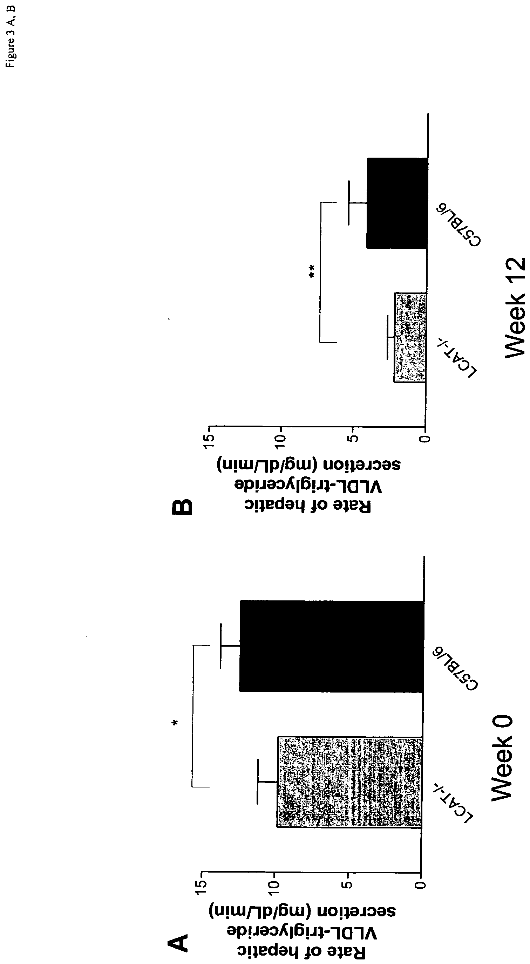

**Figure 3** shows the rate of hepatic VLDL triglyceride secretion in LCAT^{-/-} and C57BL/6 mice at weeks 0 and 12 of the experiment. Triton WR1339 (500 mg/kg body weight) was injected into 5 fasted mice per group. Serum samples were collected at 20, 40, 60, and 90 min after the injection with the detergent. As control, serum samples were isolated 1 min immediately after the injection with the detergent. Serum triglyceride levels were determined and a linear graph of serum triglyceride concentration versus time was generated. Linear regression analysis was performed and the rate of VLDL-triglyceride secretion expressed in mg/dl/min was calculated from the slope of the linear graph for each individual mouse. The bar-graph represents the mean +/- standard deviation, of the individual rates of VLDL-triglyceride secretion per virus group.

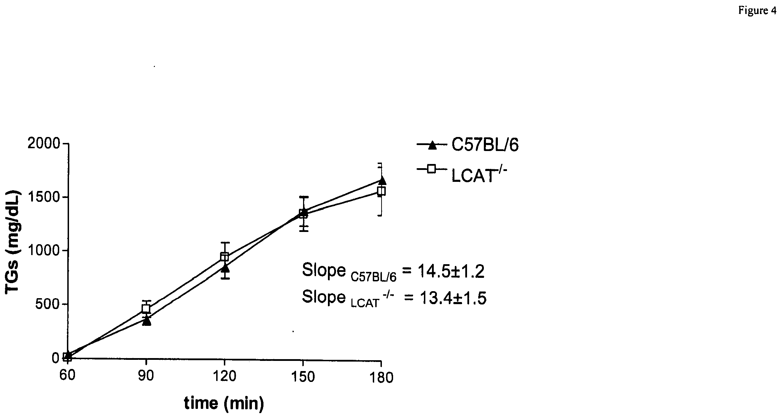

**Figure 4** shows the rate of total triglyceride input in the plasma of LCAT^{-/-} and C57BL/6 mice. Groups of 5 LCAT^{-/-} or C57BL/6 mice were fasted overnight for 16 hours. The following day animals were gavaged with 0.3 ml of olive oil and placed back to their cages for one hour. Then, mice were injected with Triton-WR1339 as described in *Methods* and serum samples were isolated at 30 min, 60 min, 90 min, 120 min, 150, and 180 min, after Triton-WR1339 injection. As control, serum samples were isolated approximately 1 min after injection with the detergent. Plasma triglyceride levels at each time-point were determined as described above, and linear graphs of triglyceride concentration *vs*. time were generated. Linear regression analysis was performed and the rate of plasma-triglyceride accumulation expressed in mg/dl/min was calculated from the slope of the linear graphs. Total plasma triglyceride supply is the sum of intestinal and hepatic triglyceride secretion.

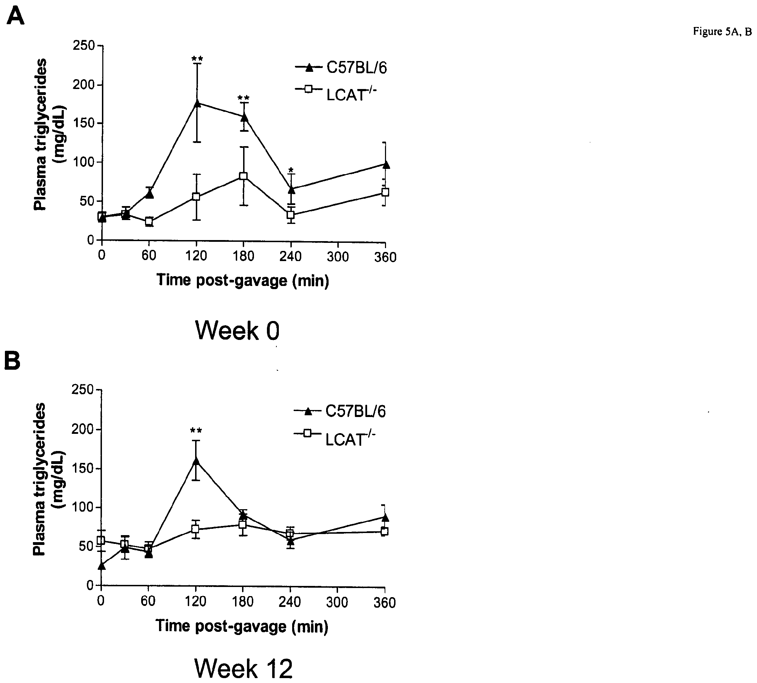

**Figure 5** shows the kinetics of post-prandial triglyceride clearance in LCAT^{-/-} and C57BL/6 mice. Groups of 5 LCAT^{-/-} or C57BL/6 mice were fasted overnight for 16 hours and the following day animals were administered an oral load of 0.5 ml of olive oil. Then, plasma samples were isolated at 30, 60, 120, 180, and 240 minutes post gavage and plasma triglyceride levels were quantified and plotted in graphs as a function of time. Values were expressed in mg/dl ± standard error of the mean.

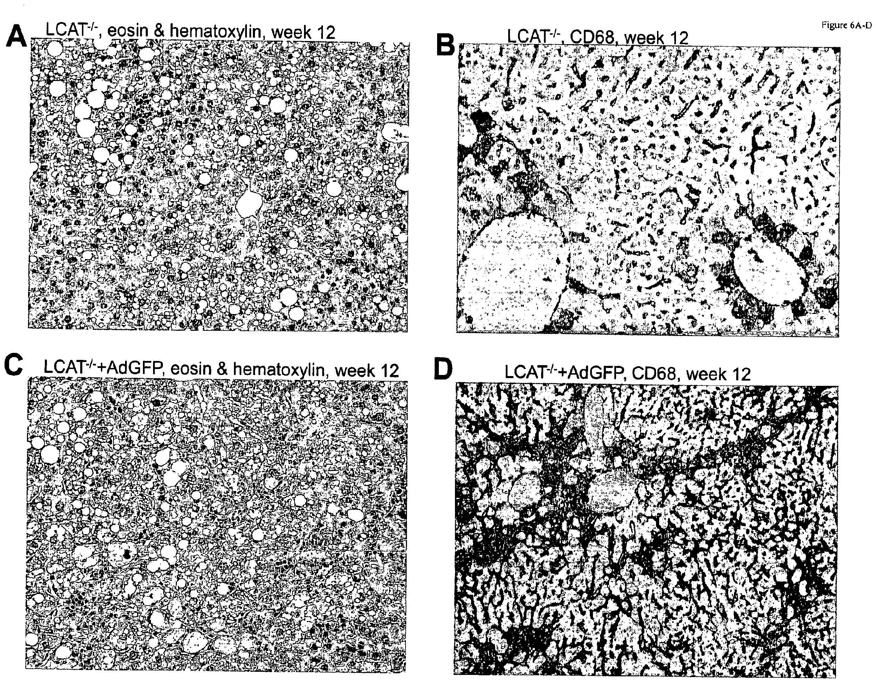

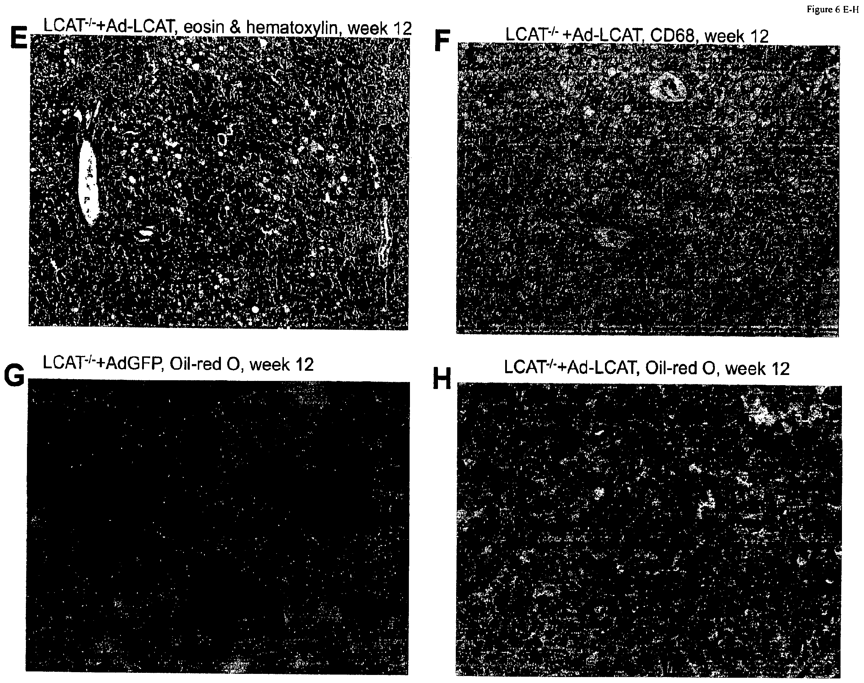

**Figure 6** shows the histological analysis of liver sections from mice infected with an LCAT-expressing or a control adenovirus. *Panels A, C, and E* are representative pictures of eosin/hematoxylin stained hepatic sections from uninfected LCAT^{-/-} mice (*A*), LCAT^{-/-} mice infected with the control AdGFP adenovirus (*C*), and LCAT^{-/-} mice infected with the Ad-LCAT adenovirus (*E*), that were fed western-type diet for 12 weeks. *Panels B, D, and F,* are representative pictures of anti-CD68 antibody stained hepatic sections from uninfected LCAT^{-/-}mice (*B*), LCAT^{-/-} mice infected with the AdGFP adenovirus (*D*), and LCAT^{-/-} mice infected with the Ad-LCAT adenovirus (*F*), that were fed western-type diet for 12 weeks. *Panels G and H* are representative pictures of oil-red O stained hepatic sections from LCAT^{-/-} mice infected with the control AdGFP (*G*) or the Ad-LCAT *(H)* adenovirus, that were fed western-type diet for 12 weeks. All pictures were taken under original magnification 40X.

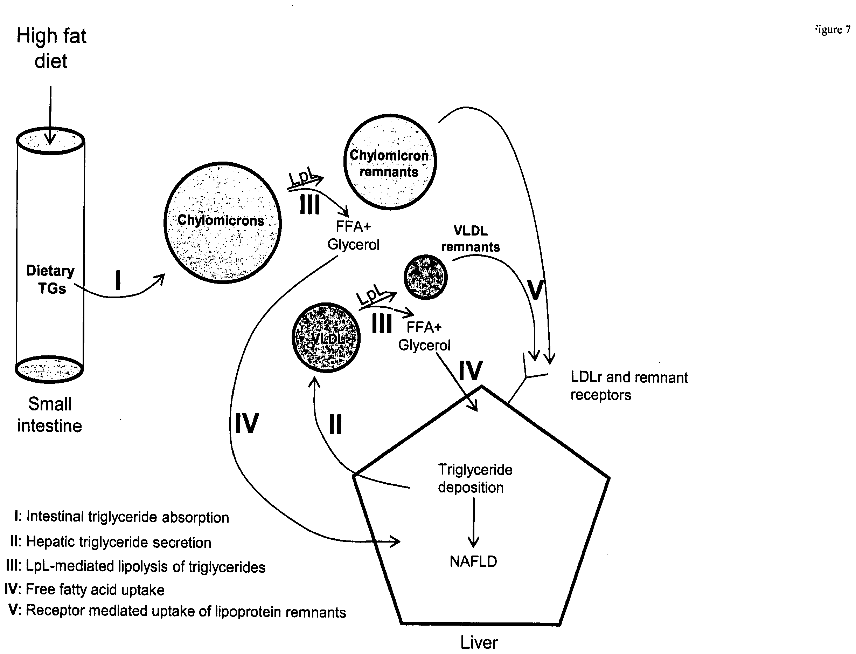

**Figure 7** shows the model summarizing the effects of LCAT deficiency on plasma lipid homeostasis and hepatic deposition of plasma triglycerides. LCAT deficiency increases intestinal absorption and secretion of triglyceride-rich chylomicrons in plasma (step I), reduces the rate of hepatic VLDL-triglyceride secretion (step II), and accelerates plasma triglyceride clearance from the circulation (steps III+V+IV), leading to enhanced deposition of lipids to the liver of LCAT^{-/-}mice.

### RESULTS

### LCAT deficiency enhances hepatic triglyceride accumulation and the development of NAFLD in mice

To test the effects of LCAT on hepatic triglyceride accumulation, groups of 10-12 week-old male LCAT-deficient (LCAT^{-/-}) and wild-type (WT) C57BL/6 mice were placed on western-type for 24 weeks. Hematoxylin and Eosin (H&E) staining of liver sections from wild-type (WT) C57BL/6 and LCAT^{-/-} mice at the beginning of the experiment (week 0) did not reveal any significant accumulation of hepatic triglycerides within the hepatocytes of these mice (Fig. 1A, E). However, at week 24, both C57BL/6 mice and LCAT^{-/-} mice showed accumulation of hepatic triglycerides in the form of lipid droplets (Fig. 1B, F). Statistical analysis following histomorphometric evaluation of the H&E sections revealed that the number of lipid droplets within hepatocytes was significantly elevated in the LCAT^{-/-} mice as compared to the C57BL/6 mice (p=0.0001) (compare Fig. IE to 1B). The observed steatosis was diffuse and of the macrovesicular type. This finding was further confirmed by oil-red O staining of hepatic sections from C57BL/6 and LCAT^{-/-} mice fed western-type diet for 24 weeks (Fig.1D, H). In agreement with these data, staining of hepatic sections with reticulin showed that in LCAT^{-/-} mice fed western-type diet for 24 weeks, NAFLD was much more progressed and resulted in a significant disruption of the normal architecture of the liver extracellular reticulin fibrils (Fig. 1G), as compared to C57BL/6 mice (Fig. 1C). No significant differences in the size and shape of visceral adipocytes were detected between the two mouse groups (data not shown).

Biochemical measurement of hepatic triglyceride content showed that LCAT^{-/-} mice fed western-type diet for 24 weeks had a triglyceride content of 197.3±10.6 mg per gram of hepatic tissue while C57BL/6 mice had a significantly lower hepatic triglyceride content (155.7±10 mg per gram of hepatic tissue, p<0.005) further confirming that LCAT possesses a central role in the deposition of triglycerides to the liver of mice and the development of diet-induced NAFLD following feeding with western-type diet.

### Body-weight of mice fed western-type diet for 24 weeks

To test the effects of LCAT deficiency on body weight gain, the weights of LCAT^{-/-} and C57BL/6 mice fed western-type diet were monitored every six weeks for a total period of 24 weeks.

As expected, C57BL/6 mice showed an increase in their body-weight during the course of the experiment (33). At week 6, C57BL/6 mice had an average body-weight of 31.8±1.7 grams (23.5±3.9% increase, as compared to their starting weight of 25.8±1 at week 0, p<0.05), at week 12 their body-weight was already 35.3±0.6 grams, while at week 24 it increased further to 42.8±1.7 grams (66.7±5.6% increase, as compared to their starting weight at week 0, p<0.05) (Fig. 2A).

Interestingly, LCAT^{-/-} mice were significantly more sensitive to the development of diet-induced obesity compared to the C57BL/6 mice. Specifically, during the course of the experiment LCAT^{-/-} mice showed a dramatic increase in body-weight (Fig. 2A). At week 6 of the experiment, the LCAT^{-/-} mouse group had an average body-weight of 32.4±1.6 grams (38.3±2.3% increase as compared to their starting weight of 23.4±0.9 grams at week 0, p<0.05). At week 12, their average body-weight was 42.2±1.1 grams while at week 24, their body-weight further increased to 50.5±1.9 grams (116.5±8.4% increase, as compared to their starting weight at week 0, p<0.05) (Fig. 2A).

To our surprise, the increased body weight, total body fat content and hepatic triglyceride content of the LCAT^{-/-} mice did not correlate with significant metabolic perturbations in these mice, compared to C57BL/6 mice. Direct comparison of insulin sensitivity and glucose tolerance between LCAT^{-/-} and C57BL/6 mice did not reveal any significant differences between the two mouse groups though LCAT^{-/-} mice were somewhat more glucose intolerant (data not shown).

### Plasma lipid levels of mice fed western-type diet for 24 weeks

To determine how plasma lipid levels in LCAT^{-/-} and C57BL/6 mice may correlate with differences in hepatic triglyceride accumulation, fasting plasma samples were isolated every 6 weeks and cholesterol, triglyceride and free-fatty acid levels were measured as described in *Materials and Methods.* C57BL/6 mice on high fat diet for 24 weeks had significantly elevated fasting cholesterol levels (224.6±21 mg/dl) as compared to their starting cholesterol levels at week 0 (91.9±10 mg/dl) (Fig. 2B), while their plasma triglyceride levels remained normal (25.1±2.9 mg/dl at week 24 vs. 18.2±1.1 mg/dl at week 0) (Fig. 2C). LCAT^{-/-} mice also showed a modest increase in their plasma cholesterol levels during the course of the experiment. At week 24 of the experiment plasma cholesterol levels of the LCAT^{-/-} mice were elevated (271.6±16.8 mg/dl at week 24 versus 88.2±20.5 mg/dl at week 0) (Fig. 2B), while their plasma triglyceride levels remained within normal range (53.2±10.3 mg/dl at week 24 versus 8.2±1.9 mg/dl at week 0, p<0.001) (Fig. 2C).

Interestingly, LCAT^{-/-} mice had slightly lower steady-state plasma FFA levels compared to C57BL/6 mice. At week 24 of the experiment plasma free fatty-acid levels of the LCAT^{-/-} mice were 1.0±0.2 mmole Equiv, while C57BL/6 mice had a concentration of 1.4±0.7 mmole Equiv (p<0.005).

Fractionation of plasma lipoproteins by density gradient ultracentrifugation followed by determination of total cholesterol, triglycerides, and phospholipids in the different density fractions revealed that deficiency in LCAT had a profound effect on the distribution and composition of plasma lipoproteins of mice fed western-type diet for 24 weeks. Specifically, in LCAT deficient mice most of the cholesterol was found in the chylomicron/VLDL and IDL density fractions while HDL cholesterol was significantly reduced (Fig. 2D). In contrast, in C57BL/6 mice the vast majority of cholesterol was distributed in the HDL density fractions while chylomicron/VLDL and IDL cholesterol levels were very low (Fig. 2D). The chylomicron/VLDL fraction of LCAT^{-/-} mice had also a much higher concentration of triglycerides consistent with the higher steady-state triglyceride levels in the plasma of these mice (Fig. 2E). As expected, most of the cholesterol of LCAT deficient mice was unesterified (data not shown).

### Rate of hepatic triglyceride secretion in LCAT^{-/-} and C57BL/6 mice

One mechanism that could affect hepatic triglyceride content is the secretion of hepatic triglycerides in the circulation. Thus, we next performed a classical VLDL triglyceride secretion assay in LCAT^{-/-} and C57BL/6 mice at weeks 0 (immediately prior to diet initiation) and 12 following feeding with western-type diet. We found that LCAT^{-/-} mice had a reduced rate of hepatic VLDL-triglyceride secretion at both time-points when compared to C57BL/6 mice. Interestingly, western-type diet for 12 weeks led to a reduction of hepatic VLDL-triglyceride secretion in both mouse strains. Specifically, at week 0, secretion rates were 9.8±1.1 mg/dl/min versus 12.5±1.3 mg/dl/min for LCAT^{-/-} and C57BL/6 mice respectively (p<0.005). At week 24, secretion rates were reduced to 2.1±0.6 mg/dl/min versus 4.2±0.9 mg/dl/ for LCAT^{-/-} and C57BL/6 mice respectively, (p<0.005) (Fig. 3).

### Rate of intestinal triglyceride secretion in LCAT^{-/-} and C57BL/6 mice.

One additional mechanism that could explain the increased sensitivity of LCAT^{-/-} mice to diet-induced NAFLD, could be increased intestinal secretion of triglyceride-rich lipoproteins to the plasma of these mice. To determine the rate of intestinal triglyceride secretion we first determined the total rate (intestinal and hepatic) of plasma triglyceride supply in LCAT^{-/-} and C57BL/6 mice fed western-type diet, following an oral fat load. Groups of 5 LCAT^{-/-} and C57BL/6 mice each were fasted for 16 hours, and then administered an oral fat load of 300 µl of olive oil, as described in *Materials and Methods.* One hour post-gavage, mice were injected with Triton WR1339 , then plasma triglyceride levels were measured as a function of time and a linear graph of plasma triglycerides versus time was created. As shown in Fig. 4, both mouse strains exhibited comparable rates of plasma triglyceride increase (14.5±1.2 mg/dl/min for C57BL/6 vs. 13.4±1.5 mg/dl/min for LCAT^{-/-} mice, p>0.05) as determined from the slopes of the graphs.

Then, by subtracting the rate of hepatic triglyceride secretion (determined above) from the total rate of plasma triglyceride supply, the rate of intestinal triglyceride secretion at week 0 of the experiment was determined as 3.6±0.5 mg/dl/min for the LCAT^{-/-} mice and 2.0±0.7 mg/dl/min for the C57BL/6 mice (p<0.05).

No significant differences in the average daily food consumption were found between the two mouse strains. Specifically, two independent measurements, one at week 12 and one at week 24, showed that LCAT^{-/-} mice consumed 3.6±0.1 and 3.6±0.3 grams/mouse/day respectively (p>0.05) while C57BL/6 mice consumed 3.8±0.2 and 3.4±0.2 grams/mouse/day (p>0.05) respectively (measurements represent an average daily consumption during a 7 day period). No statistical difference was observed between the two mouse strains at each time-point (p>0.05).

### Kinetics of post-prandial triglyceride clearance in LCAT^{-/-} and C57BL/6 mice

Another potential mechanism that could explain the increased sensitivity of LCAT^{-/-} mice to diet-induced NAFLD, could be increased clearance of plasma triglycerides in these mice. To determine the kinetics of post-prandial triglyceride clearance, groups of five LCAT^{-/-} and five C57BL/6 mice were fasted for 16 hours, then administered an oral fat load of 300 µl of olive oil and plasma samples were isolated at 30, 60, 120, 180, 240 and 360 min post gavage. As shown in Fig. 5A, plasma triglyceride levels in LCAT^{-/-} mice started increasing at 120 min (56.3±29.5 mg/dl), reached a modest peak at 180 min (83.5±37.4 mg/dl) and rapidly declined back to baseline by 240 min (34.2±10.5 mg/dl). In contrast, control C57BL/6 mice showed a much slower kinetics of catabolism of postprandial triglycerides. Specifically, plasma triglyceride levels in C57BL/6 started increasing as early as 60 min (61.6±6.6 mg/dl) post-gavage. At 120 and 180 min, plasma triglyceride levels were 177.4±50.8 mg/dl and 159.7±18.3 mg/dl respectively, and then started declining slowly, reaching a concentration of 67.7±19.5 mg/dl at 180 min of the experiment (Fig. 5A). Similar post-prandial triglyceride kinetics was obtained for LCAT^{-/-} and C57BL/6 mice fed western-type diet for 12 weeks (Fig. 5B). Given the enhanced intestinal absorption of the LCAT^{-/-} mice, the data support that these mice also display a very fast kinetics of post-prandial triglyceride clearance.

### Ectopic expression of LCAT by adenovirus-mediated gene transfer reduces hepatic triglyceride content in LCAT^{-/-} mice fed western-type diet for 12 weeks

In the next set of experiments we investigated the effects of ectopic overexpression of LCAT on hepatic triglyceride deposition and NAFLD. Three groups of six LCAT^{-/-} mice each were formed and fed western-type diet for 12 weeks. At the end of this period, one group received 8x10⁸ pfu of Ad-LCAT, the second group received 8x10⁸ pfu of a control adenovirus AdGFP, while the third group remained uninfected. Immediately after infection, all three groups of mice were switched to standard chow diet for an additional 10 days. Then, mice were sacrificed and liver samples were collected for histological and biochemical analyses. Western blot analysis of plasma samples from infected mice confirmed that only mice infected with the Ad-LCAT adenovirus were expressing LCAT in the circulation (data not shown).

As expected, H&E staining of livers from uninfected LCAT^{-/-} mice fed western-type diet for 12 weeks reveal significant accumulation of triglycerides in the hepatocytes of these mice. Notably, the observed steatosis was of the microvesicular type (Fig. 6A). Similarly LCAT^{-/-} mice infected with 8x10⁸ pfu of the control adenovirus AdGFP showed a comparable accumulation of hepatic triglycerides and some signs of inflammation possibly due to the infection process (Fig. 6C). However, LCAT^{-/-} mice infected with 8x10⁸ pfu of the Ad-LCAT adenovirus had a profoundly reduced deposition of hepatic triglycerides and exhibited a significantly improved hepatic histology (Fig. 6E). Oil-red O staining (Fig. 6 G, H) further confirmed that LCAT^{-/-} mice treated with the Ad-LCAT adenovirus (Fig. 6H) had significantly reduced hepatic lipid content compared to the LCAT^{-/-} mice treated with the control adenovirus AdGFP (Fig. 6G). Immunohistochemical staining of hepatic sections with an anti-CD68 antibody revealed that both uninfected and AdGFP-infected LCAT^{-/-} mice exhibited increased infiltration of macrophages, residing mainly around the hepatic venules and the lipid loaded hepatocytes (Fig. B, D). Control AdGFP-infected LCAT^{-/-} mice showed the highest infiltration of macrophages, possibly due to an inflammatory response associated with the infection process. Interestingly, treatment of LCAT^{-/-} mice with Ad-LCAT adenovirus significantly reduced the number of infiltrated macrophages accumulating in the liver of these mice (compare Fig. 6F to Fig. 6B, D). Histomorphometry followed by statistical analysis showed that the number of lipid droplets was significantly reduced in the livers of LCAT^{-/-} mice infected with the LCAT-expressing adenovirus as compared to the livers of uninfected or the control AdGFP adenovirus-infected LCAT^{-/-} mice (p=0.0001).

In agreement with these data, biochemical determination of hepatic triglyceride content showed that LCAT^{-/-} mice treated with the control adenovirus had a triglyceride content of 121.2±5.9 mg of glycerol equivalents per gram of tissue, while LCAT^{-/-} mice treated with Ad-LCAT had a significantly lower triglyceride content of 95.1±5.8 mg of glycerol equivalents per gram of tissue (p<0.05). Taken together, the findings establish that ectopic expression of LCAT results in a significant reduction of hepatic triglyceride content and a great improvement of hepatic histology and architecture in LCAT^{-/-} mice fed western-type diet for 12 weeks.

### Detailed description of the invention

LCAT is an important plasma enzyme of the lipoprotein transport system, being responsible for the esterification of the free cholesterol of lipoproteins (12-15) and their proper metabolism in plasma (16-23;23;25;26). In the present work we describe that LCAT affects diet-induced hepatic triglyceride accumulation and NAFLD. We tested this hypothesis in experimental mouse models. In our studies we focused on diet-induced hepatic triglyceride accumulation, since most reported cases of NAFLD are due to an imbalance in caloric load (4).

To this date, the vast majority of the studies published in the literature have focused almost exclusively on the effects of LCAT expression on fasting plasma lipid levels and atherosclerosis, while the involvement of LCAT in the metabolism of dietary triglycerides and hepatic triglyceride deposition has not been investigated so far. The present invention is the first study to investigate the role of LCAT in these processes and its relationship to NAFLD. We report that LCAT deficiency sensitizes mice towards hepatic triglyceride accumulation and development of NAFLD while adenovirus-mediated expression of LCAT improves significantly hepatic histology and architecture in the treated mice.

Histological evaluation of the liver samples from the two mouse groups using H&E and oil-red O staining revealed increased levels of steatosis in the LCAT^{-/-} mice (Fig. 1F, H), as demonstrated by the existence of a large number of lipid droplets within the vast majority of the examined hepatocytes. Steatosis was diffuse and of the macrovesicular type, in which a large fat vacuole within the hepatocyte pushed the nucleus towards the edge of the cell. In contrast, liver sections from C57BL/6 mice, had significantly less steatosis and there was evidence of reduced lipid accumulation within hepatocytes (Fig. 1B, D). Our histological findings were in harmony with the results obtained by the reticulin stain showing that in the livers of C57BL/6 mice the reticulin network was minimally distorted, in contrast to livers of LCAT^{-/-} mice that were heavily loaded with fat (Fig. 1C, G).

In an attempt to provide a mechanistic interpretation to our histological findings, we studied the effects of LCAT deficiency on plasma triglyceride metabolism. Our kinetic analysis indicates that deficiency in LCAT enhances intestinal absorption and secretion of triglycerides in the plasma of experimental mice (Fig. 7, step I) while it reduces significantly the secretion of triglyceride-rich VLDL from the liver (Fig. 7, step II).

Additional kinetic analyses showed that LCAT^{-/-} mice also exhibit a much more efficient catabolism of post-prandial triglyceride-rich lipoproteins (TRLs) as determined by the rate of clearance of plasma triglycerides from the circulation, following an oral administration of fat load (Fig. 4A, B). Therefore, in addition to the enhanced intestinal absorption and reduced hepatic triglyceride secretion, LCAT deficiency also promotes the more efficient deposition of dietary triglycerides to the liver and possibly other peripheral tissues (Fig. 7, steps IV+V). This conclusion is further supported by the observation that LCAT^{-/-} mice become more obese than C57BL/6 mice. It is possible that LCAT confers, directly or indirectly, alterations in the structure and composition of TRLs, which in turn may affect their rate of clearance. Direct effects may be related to the cholesteryl-ester load of TRLs which may dictate their size, structure and apoprotein composition. Indirect effects may relate to a potential LCAT-mediated cross-talk between HDL and TRLs which may affect the lipid and apoprotein composition of the TRLs, and their subsequent capacity to deliver dietary lipids to the liver and other tissues. A similar ABCA1-mediated cross-talk between HDL and TRLs that modulates TRL structure and clearance and subsequently plasma triglyceride levels has been recently reported by us (29).

Analysis of plasma cholesterol and triglyceride levels at week 24 showed that LCAT^{-/-}mice had slightly lower cholesterol levels and significantly higher triglyceride levels compared to C57BL/6 mice (Fig. 2B, C). Fractionation of plasma lipoproteins by density gradient ultracentrifugation followed by measurement of total cholesterol and triglycerides in the different fractions indicated that deficiency in LCAT has a profound effect on the distribution and composition of plasma lipoproteins of mice fed western-type diet for 24 weeks (Fig. 2D, E). In LCAT^{-/-} mice cholesterol was mainly found in the chylomicron/VLDL/IDL fractions (these mice do not have significant levels of circulating LDL), while the majority of plasma cholesterol of C57BL/6 mice was in the HDL. The chylomicron/VLDL/IDL fractions of LCAT^{-/-} mice had also higher triglyceride content, a finding consistent with the increased intestinal absorption of dietary lipids in these mice.

Although previous studies suggested that elevated plasma free fatty acids are pivotal in NAFLD development (38), in our experiments LCAT^{-/-} mice fed western type diet had lower plasma free fatty-acid levels and higher plasma triglyceride levels than C57BL/6 mice. This observation is consistent with our previous finding that LCAT potentiates the activity of lipoprotein lipase, resulting in enhanced lipolysis of triglyceride-rich lipoproteins in plasma (26). Nevertheless, it is possible that free fatty acids may have some contribution to hepatic triglyceride content in LCAT^{-/-} mice (Fig.7, step IV), though the extent of this contribution may be low.

To explore the therapeutic potential of LCAT in the treatment of NAFLD, as a proof of principle we used adenovirus-mediated gene transfer in LCAT^{-/-} mice fed western-type diet for a period of 12 weeks. We chose to administer a low dose of 8x10⁸ pfu of adenovirus in order to avoid infection-induced inflammation and liver damage that could affect the outcome of our experiment. The inclusion of the AdGFP-infected group was necessary in order to correct for any non-specific effects due to the infection process. Following infection with the adenoviruses, all three mouse groups were switched to chow-diet for 10 days, and then hepatic triglyceride content and tissue architecture were determined. Ectopic expression of LCAT resulted in a significantly reduced triglyceride content in the livers of these mice, and an improved hepatic histology, as compared to mice infected with the control AdGFP adenovirus or the non-infected mice. It is interesting that only 10 days of LCAT expression were sufficient to improve the histopathological condition of the livers of these mice. The natural ability of the liver to regenerate may play a significant role in the improvement of hepatic histology following ectopic expression of LCAT. It is possible that in the absence of dietary fat ingestion, adenovirus-mediated expression of LCAT increased the rate of hepatic triglyceride secretion in the plasma of these mice. However, additional parameters such as hepatic triglyceride synthesis or hepatic β-oxidation of fatty acids may also be affected by LCAT overexpression though no mechanistic relationship between LCAT and these processes has been previously documented in the literature.

In our studies, LCAT deficient mice are also more sensitive that control C57BL/6 mice to diet-induced obesity though they did not develop insulin resistance, glucose intolerance or other obesity-related metabolic perturbations. Histological analysis did not reveal any significant differences in the size of adipocytes in the epididymal fat isolated from LCAT^{-/-} and C57BL/6 mice (data not shown), a finding that may explain the lack of significant metabolic abnormalities in the more obese LCAT^{-/-} mice. Considering that obesity-related metabolic disorders develop over a long period of time, it is plausible that insulin resistance and glucose intolerance would have developed, had the experiment progressed for a longer period of time.

Of note, more than three decades ago Sabesin and coworkers (39) suggested that in patients with alcoholic liver disease, chronic alcohol abuse correlated with a significant reduction in plasma LCAT activity, as it was manifested by the reduced levels of plasma cholesteryl esters, increased levels of plasma unesterified cholesterol, and striking alterations in the structure and electrophoretic mobility of plasma HDL in these patients. By revealing the importance of plasma LCAT activity on hepatic triglyceride deposition and NAFLD, our data also provide a mechanistic interpretation for the alcoholic hepatic steatosis seen in patients with alcoholic liver disease.

In addition to its already well-established role in lipoprotein metabolism and atherosclerosis, our results identify for the first time LCAT as an important link between lipoprotein metabolism and hepatic triglyceride accumulation, while they provide a mechanistic interpretation for the concurrence of dyslipidemia and nonalcoholic fatty liver disease in patients with metabolic syndrome. Overall, the findings of the present study extend significantly beyond the current state-of-the-art in relation to the functions of LCAT and claim that that LCAT activity is an effective therapeutic target in the treatment of this pathological condition.

### REFERENCES

(1) Preiss, D., Sattar, N. (2008) Non-alcoholic fatty liver disease: an overview of prevalence, diagnosis, pathogenesis and treatment considerations. Clin.Sci.(Lond) 115, 141-150
(2) Angulo, P. (2002) Nonalcoholic fatty liver disease. N.Engl.J.Med. 346, 1221-1231
(3) Chitturi, S., Farrell, G. C. (2001) Etiopathogenesis of nonalcoholic steatohepatitis. Semin.Liver Dis. 21, 27-41
(4) Browning, J. D., Horton, J. D. (2004) Molecular mediators of hepatic steatosis and liver injury. J.Clin.Invest 114, 147-152
(5) Hamaguchi, M., Kojima, T., Takeda, N., Nakagawa, T., Taniguchi, H., Fujii, K., Omatsu, T., Nakajima, T., Sarui, H., Shimazaki, M., Kato, T., Okuda, J., Ida, K. (2005) The metabolic syndrome as a predictor of nonalcoholic fatty liver disease. Ann.Intern.Med. 143, 722-728
(6) Fan, J. G., Zhu, J., Li, X. J., Chen, L., Lu, Y. S., Li, L., Dai, F., Li, F., Chen, S. Y. (2005) Fatty liver and the metabolic syndrome among Shanghai adults. J.Gastroenterol.Hepatol. 20, 1825-1832
(7) Browning, J. D., Szczepaniak, L. S., Dobbins, R., Nuremberg, P., Horton, J. D., Cohen, J. C., Grundy, S. M., Hobbs, H. H. (2004) Prevalence of hepatic steatosis in an urban population in the United States: impact of ethnicity. Hepatology 40, 1387-1395
(8) Marchesini, G., Brizi, M., Bianchi, G., Tomassetti, S., Bugianesi, E., Lenzi, M., McCullough, A. J., Natale, S., Forlani, G., Melchionda, N. (2001) Nonalcoholic fatty liver disease: a feature of the metabolic syndrome. Diabetes 50, 1844-1850
(9) Byrne, C. D., Olufadi, R., Bruce, K. D., Cagampang, F. R., Ahmed, M. H. (2009) Metabolic disturbances in non-alcoholic fatty liver disease. Clin.Sci.(Lond) 116, 539-564
(10) Cortes, V. A., Curtis, D. E., Sukumaran, S., Shao, X., Parameswara, V., Rashid, S., Smith, A. R., Ren, J., Esser, V., Hammer, R. E., Agarwal, A. K., Horton, J. D., Garg, A. (2009) Molecular mechanisms of hepatic steatosis and insulin resistance in the AGPAT2-deficient mouse model of congenital generalized lipodystrophy. Cell Metab 9, 165-176
(11) Tirosh, O., Artan, A., Aharoni-Simon, M., Ramadori, G., Madar, Z. (2010) Impaired liver glucose production in a murine model of steatosis and endotoxemia: protection by inducible nitric oxide synthase. Antioxid.Redox.Signal. 13, 13-26
(12) Simon, J. B., Boyer, J. L. (1970) Production of lecithin: cholesterol acyltransferase by the isolated perfused rat liver. Biochim.Biophys.Acta 218, 549-551
(13) Osuga, T., Portman, O. W. (1971) Origin and disappearance of plasma lecithin: cholesterol acyltransferase. Am.J.Physiol 220, 735-741
(14) Soutar, A. K., Garner, C. W., Baker, H. N., Sparrow, J. T., Jackson, R. L., Gotto, A. M., Smith, L. C. (1975) Effect of the human plasma apolipoproteins and phosphatidylcholine acyl donor on the activity of lecithin: cholesterol acyltransferase. Biochemistry 14, 3057-3064
(15) Santamarina-Fojo, S., Hoeg, J. M., Assmann, G., Brewer, H. B., Jr. (2001) Lecithin cholesterol acyltransferase deficiency and fish eye disease. In The Metabolic & Molecular Bases of Inherited Disease (Scriver, C. R., Beaudet, A. L., Sly, W. S., and Valle, D., eds) pp. 2817-2834, McGraw-Hill, New York.
(16) Norum, K. R., Gjone, E. (1967) Familial serum-cholesterol esterification failure. A new inborn error of metabolism. Biochim.Biophys.Acta 144, 698-700
(17) Norum, K. R., Gjone, E. (1968) The effect of plasma transfusion on the plasma cholesterol esters in patients with familial plasma lecithin: cholesterol acyltransferase deficiency. Scand.J.Clin.Lab Invest 22, 339-342
(18) Gjone, E., Norum, K. R. (1968) [Familial plasma cholesterol ester deficiency. A new inborn error of metabolism]. Nord.Med. 80, 878-883
(19) Torsvik, H., Gjone, E., Norum, K. R. (1968) Familial plasma cholesterol ester deficiency. Clinical studies of a family. Acta Med.Scand. 183, 387-391
(20) Gjone, E., Norum, K. R. (1968) Familial serum cholesterol ester deficiency. Clinical study of a patient with a new syndrome. Acta Med.Scand. 183, 107-112
(21) Hamnstrom, B., Gjone, E., Norum, K. R. (1969) Familial plasma lecithin: cholesterol acyltransferase deficiency. Br.Med.J. 2, 283-286
(22) Norum, K. R., Borsting, S., Grundt, I. (1970) Familial lecithin: cholesterol acyltransferase deficiency. Study of two new patients and their close relatives. Acta Med.Scand. 188, 323-326
(23) Glomset, J. A., Norum, K. R., King, W. (1970) Plasma lipoproteins in familial lecithin: cholesterol acyltransferase deficiency: lipid composition and reactivity in vitro. J.Clin.Invest 49, 1827-1837
(24) Schaefer, E. J., Santos, R. D., Asztalos, B. F. (2010) Marked HDL deficiency and premature coronary heart disease. Curr.Opin.Lipidol. 21, 289-297
(25) Forte, T., Norum, K. R., Glomset, J. A., Nichols, A. V. (1971) Plasma lipoproteins in familial lecithin: cholesterol acyltransferase deficiency: structure of low and high density lipoproteins as revealed by elctron microscopy. J.Clin.Invest 50, 1141-1148
(26) Kypreos, K. E., Zannis, V. I. (2007) Pathway of biogenesis of apolipoprotein E-containing HDL in vivo with the participation of ABCA1 and LCAT. Biochem.J. 403, 359-367
(27) Brousseau, M. E., Kauffman, R. D., Herderick, E. E., Demosky, S. J., Jr., Evans, W., Marcovina, S., Santamarina-Fojo, S., Brewer, H. B., Jr., Hoeg, J. M. (2000) LCAT modulates atherogenic plasma lipoproteins and the extent of atherosclerosis only in the presence of normal LDL receptors in transgenic rabbits. Arterioscler.Thromb.Vasc.Biol. 20, 450-458
(28) Sakai, N., Vaisman, B. L., Koch, C. A., Hoyt, R. F., Jr., Meyn, S. M., Talley, G. D., Paiz, J. A., Brewer, H. B., Jr., Santamarina-Fojo, S. (1997) Targeted disruption of the mouse lecithin:cholesterol acyltransferase (LCAT) gene. Generation of a new animal model for human LCAT deficiency. J.Biol.Chem. 272, 7506-7510
(29) Kypreos, K. E. (2008) ABCA1 Promotes the de Novo Biogenesis of Apolipoprotein CIII-Containing HDL Particles in Vivo and Modulates the Severity of Apolipoprotein CIII-Induced Hypertriglyceridemia. Biochemistry 47, 10491-10502
(30) Kypreos, K. E., Teusink, B., Van Dijk, K. W., Havekes, L. M., Zannis, V. I. (2001) Analysis of the structure and function relationship of the human apolipoprotein E in vivo, using adenovirus-mediated gene transfer. FASEB J. 15, 1598-1600
(31) Papachristou, D. J., Papadakou, E., Basdra, E. K., Baltopoulos, P., Panagiotopoulos, E., Papavassiliou, A. G. (2008) Involvement of the p38 MAPK-NF-kappaB signal transduction pathway and COX-2 in the pathobiology of meniscus degeneration in humans. Mol.Med. 14, 160-166
(32) Duivenvoorden, I., Teusink, B., Rensen, P. C., Romijn, J. A., Havekes, L. M., Voshol, P. J. (2005) Apolipoprotein C3 deficiency results in diet-induced obesity and aggravated insulin resistance in mice. Diabetes 54, 664-671
(33) Karagiannides, I., Abdou, R., Tzortzopoulou, A., Voshol, P. J., Kypreos, K. E. (2008) Apolipoprotein E predisposes to obesity and related metabolic dysfunctions in mice. FEBS J.
(34) Aalto-Setala, K., Fisher, E. A., Chen, X., Chajek-Shaul, T., Hayek, T., Zechner, R., Walsh, A., Ramakrishnan, R., Ginsberg, H. N., Breslow, J. L. (1992) Mechanism of hypertriglyceridemia in human apolipoprotein (apo) CIII transgenic mice. Diminished very low density lipoprotein fractional catabolic rate associated with increased apo CIII and reduced apo E on the particles. J.Clin.Invest 90, 1889-1900
(35) Kypreos, K. E., Van Dijk, K. W., van Der, Z. A., Havekes, L. M., Zannis, V. I. (2001) Domains of apolipoprotein E contributing to triglyceride and cholesterol homeostasis in vivo. Carboxyl-terminal region 203-299 promotes hepatic very low density lipoprotein-triglyceride secretion. J.Biol.Chem. 276, 19778-19786
(36) Kypreos, K. E., Van Dijk, K. W., Havekes, L. M., Zannis, V. I. (2005) Generation of a recombinant apolipoprotein E variant with improved biological functions: hydrophobic residues (LEU-261, TRP-264, PHE-265, LEU-268, VAL-269) of apoE can account for the apoE-induced hypertriglyceridemia. J.Biol.Chem. 280, 6276-6284
(37) Kypreos, K. E., Karagiannides, I., Fotiadou, E. H., Karavia, E. A., Brinkmeier, M. S., Giakoumi, S. M., Tsompanidi, E. M. (2009) Mechanisms of obesity and related pathologies: Role of apolipoprotein E in the development of obesity. FEBS J
(38) Malhi, H., Gores, G. J. (2008) Molecular mechanisms of lipotoxicity in nonalcoholic fatty liver disease. Semin.Liver Dis. 28, 360-369
(39) Sabesin, S. M., Hawkins, H. L., Kuiken, L., Ragland, J. B. (1977) Abnormal plasma lipoproteins and lecithin-cholesterol acyltransferase deficiency in alcoholic liver disease. Gastroenterology 72, 510-518

## Claims

1. Naturally occurring polypeptide lecithin:cholesterol acyltransferase (LCAT) for use in a method for treating nonalcoholic fatty liver disease (NAFLD) in a mammal, said method comprising administering said naturally occurring polypeptide lecithin: cholesterol acyltransferase (LCAT) to said mammal.

2. Naturally occurring polypeptide lecithin:cholesterol acyltransferase (LCAT) for use according to claim 1, wherein said polypeptide is administered by intravenous administration.

3. Naturally occurring polypeptide lecithin:cholesterol acyltransferase (LCAT) for use according to claim 1, wherein said polypeptide is administered in the form of proteoliposomes.

4. A nucleic acid encoding the human LCAT protein, operably linked to a promoter that, when expressed in the target cells, is capable of expressing said therapeutic naturally occurring human LCAT protein, for use in a method for treating nonalcoholic fatty liver disease in a mammal, said method comprising administering to said mammal an effective amount of said nucleic acid.

5. A nucleic acid encoding the human LCAT protein for use according to claim 4, wherein said nucleic acid is associated with a liposome.

6. A nucleic acid encoding the human LCAT protein for use according to claim 4, wherein said nucleic acid is administered by intravenous injection.

7. A nucleic acid encoding the human LCAT protein for use according to claim 4, wherein said nucleic acid comprises a recombinant viral vector.

8. A nucleic acid encoding the human LCAT protein for use according to claim 7, wherein said vector is an adeno-associated vector, a lentiviral vector, a herpes viral vector, or a retroviral vector.

9. A nucleic acid encoding the human LCAT protein for use according to claim 4, wherein said nucleic acid is administered to the liver.

10. Naturally occurring polypeptide lecithin:cholesterol acyltransferase (LCAT) for use according to any one of claims 1 to 3, wherein said mammal is a human.

11. A nucleic acid encoding the human LCAT protein for use according to any one of claims 4 to 9, wherein said mammal is a human.

12. Naturally occurring polypeptide lecithin:cholesterol acyltransferase (LCAT) for use according to claim 1, wherein said polypeptide is of human, murine, bovine, rat, rabbit, pigeon, hamster and monkey origin.

13. A nucleic acid encoding the human LCAT protein for use according to claim 4, wherein said nucleic acid is of human, murine, bovine, rat, rabbit, pigeon, hamster and monkey origin.

## Patentansprüche

1. Natürlich vorkommendes Polypeptid Lecithin-Cholesterin-Acyltransferase (LCAT) zur Verwendung in einem Verfahren zur Behandlung von nichtalkoholischer Fettlebererkrankung (NAFLD) bei einem Säugetier, wobei das Verfahren das Verabreichen des natürlich vorkommenden Polypeptids Lecithin-Cholesterin-Acyltransferase (LCAT) an das Säugetier umfasst.

2. Natürlich vorkommendes Polypeptid Lecithin-Cholesterin-Acyltransferase (LCAT) zur Verwendung nach Anspruch 1, wobei das Polypeptid durch intravenöse Verabreichung verabreicht wird.

3. Natürlich vorkommendes Polypeptid Lecithin-Cholesterin-Acyltransferase (LCAT) zur Verwendung nach Anspruch 1, wobei das Polypeptid in Form von Proteoliposomen verabreicht wird.

4. Nukleinsäure, die das menschliche LCAT-Protein kodiert und operabel an einen Promotor gebunden ist, der, wenn er in den Zielzellen exprimiert wird, in der Lage ist, das therapeutische natürlich vorkommende menschliche LCAT-Protein zu exprimieren, zur Verwendung in einem Verfahren zur Behandlung von nichtalkoholischer Fettlebererkrankung in einem Säugetier, wobei das Verfahren die Verabreichung einer wirksamen Menge der Nukleinsäure an das Säugetier umfasst.

5. Nukleinsäure, die das menschliche LCAT-Protein für die Verwendung nach Anspruch 4 kodiert, wobei die Nukleinsäure mit einem Liposom assoziiert ist.

6. Nukleinsäure, die das menschliche LCAT-Protein für die Verwendung nach Anspruch 4 kodiert, wobei die Nukleinsäure durch intravenöse Injektion verabreicht wird.

7. Nukleinsäure, die das menschliche LCAT-Protein für die Verwendung nach Anspruch 4 kodiert, wobei die Nukleinsäure einen rekombinanten viralen Vektor umfasst.

8. Nukleinsäure, die das menschliche LCAT-Protein für die Verwendung nach Anspruch 7 kodiert, wobei der Vektor ein adeno-assoziierter Vektor, ein lentiviraler Vektor, ein Herpes-Virusvektor oder ein retroviraler Vektor ist.

9. Nukleinsäure, die das menschliche LCAT-Protein für die Verwendung nach Anspruch 4 kodiert, wobei die Nukleinsäure an die Leber verabreicht wird.

10. Natürlich vorkommendes Polypeptid Lecithin-Cholesterin-Acyltransferase (LCAT) zur Verwendung nach irgend einem der Ansprüche 1 bis 3, wobei das Säugetier ein Mensch ist.

11. Nukleinsäure, die das menschliche LCAT-Protein für die Verwendung nach irgend einem der Ansprüche 4 bis 9 kodiert, wobei das Säugetier ein Mensch ist.

12. Natürlich vorkommendes Polypeptid Lecithin-Cholesterin-Acyltransferase (LCAT) zur Verwendung nach Anspruch 1, wobei das Polypeptid menschlichen, murinen, bovinen, Ratten-, Kaninchen-, Tauben-, Hamster- und Affen-Ursprungs ist.

13. Nukleinsäure, die das menschliche LCAT-Protein für die Verwendung nach Anspruch 4 kodiert, wobei die Nukleinsäure menschlichen, murinen, bovinen, Ratten-, Kaninchen-, Tauben-, Hamster- und Affen-Ursprungs ist.

## Revendications

1. Lécithine/cholestérol acyltransférase (LCAT) polypeptide naturel pour utilisation dans un procédé pour traiter une stéatose hépatique non alcoolique (NAFLD) chez un mammifère, ledit procédé comprenant l'administration dudit lécithine/cholestérol acyltransférase (LCAT) polypeptide naturel au dit mammifère.

2. Lécithine/cholestérol acyltransférase (LCAT) polypeptide naturel pour utilisation selon la revendication 1, dans lequel ledit polypeptide est administré par administration intraveineuse.

3. Lécithine/cholestérol acyltransférase (LCAT) polypeptide naturel pour utilisation selon la revendication 1, dans lequel ledit polypeptide est administré sous la forme de proétoliposomes.

4. Acide nucléique codant la protéine LCAT humaine, fonctionnellement liée à un promoteur qui, lorsqu'il est exprimé dans les cellules cibles, est capable d'exprimer ladite protéine LCAT humaine naturelle thérapeutique, pour utilisation dans un procédé pour traiter une stéatose hépatique non alcoolique chez un mammifère, ledit procédé comprenant l'administration au dit mammifère d'une quantité efficace dudit acide nucléique.

5. Acide nucléique codant la protéine LCAT humaine pour utilisation selon la revendication 4, dans lequel ledit acide nucléique est associé à un liposome.

6. Acide nucléique codant la protéine LCAT humaine pour utilisation selon la revendication 4, dans lequel ledit acide nucléique est administré par injection intraveineuse.

7. Acide nucléique codant la protéine LCAT humaine pour utilisation selon la revendication 4, dans lequel ledit acide nucléique comprend un vecteur viral recombinant.

8. Acide nucléique codant la protéine LCAT humaine pour utilisation selon la revendication 7, dans lequel ledit vecteur est un vecteur adéno-associé, un vecteur lentiviral, un vecteur viral de l'herpès ou un vecteur rétroviral.

9. Acide nucléique codant la protéine LCAT humaine pour utilisation selon la revendication 4, dans lequel ledit acide nucléique est administré au foie.

10. Lécithine/cholestérol acyltransférase (LCAT) polypeptide naturel pour utilisation selon chaqu'une quelconque des revendications 1 à 3, ou ledit mammifère est un humain.

11. Acide nucléique codant la protéine LCAT humaine pour utilisation selon chaqu'une des revendications 4 à 9, ou ledit mammifère est un humain.

12. Lécithine/cholestérol acyltransférase (LCAT) polypeptide naturel pour utilisation selon la revendication 1, ou ledit polypeptide est produit par les hommes, murines, bovins, rats, lapins, pigeons, hamsters et singes.

13. Acide nucléique codant la protéine LCAT humaine pour utilisation selon la revendication 4, dans lequel ledit acide nucléique est produit par les hommes, murines, bovins, rats, lapins, pigeons, hamsters et singes.
